# EUROPEAN PATENT APPLICATION

(11) **EP 1 348 447 A1**
(43) Date of publication of application: **01.10.2003**
(21) Application number: 03004200.6
(22) Date of filing: 27.02.2003
(51) Int. Cl.: A61L 9/12, A61L 9/04, B60H 3/00

(54) **Deodorising-perfuming device for motor vehicles**

(30) Priority: 18.03.2002 IT MI20020145 U
(71) Applicant: ELIOCELL S.R.L., 20019 Settimo Milanese Milano (IT)
(72) Inventor: Altmann, Fritz, 20100 Milano (IT)
(74) Representative: Coloberti, Luigi

(57) **Abstract**

Deodorising-perfuming device applicable to an internal air vent of a vehicle; the device comprises an outer casing (10) having at least one rear inlet aperture (12), and at least one outlet aperture (13) defining a flow path for an air stream. An open container (14) for a deodorising and/or perfuming gel substance (15) in a solid form, is disposed inside the outer casing (10); a manually operable control means allows a gradual withdrawal of the deodorising/perfuming gel substance (15) from the inner container (14) for its progressive exposure to the air flow.

## Description

### BACKGROUND OF THE INVENTION

This invention concerns a device for diffusing a deodorising and/or perfuming substance in the passenger compartment of a vehicle, such a car, or for any similar use.

### STATE OF THE ART

Passenger compartments of vehicles very often have a disagreeable smell, both when they are new, and during the use of the vehicles themselves. In particular, when a vehicle is new, the synthetic materials by which the internal components and upholstery are made, the adhesives and lubricants used for their assembly, give off chemical substances having a persistent odour which may prove to be unpleasant for the passengers.

During the working life of the vehicle, on the contrary, the passenger compartment develops odours, due for example to stagnant dampness, cigarette smoke, pollution and the like.

Consequently, in order to ensure greater comfort for the occupants of a vehicle, it become necessary to deodorise the passenger compartment, by diffusing a pleasant fragrance.

For this purpose, use is sometimes made of deodorants comprising a support which naturally diffuses perfumed essences; these types of deodorants however initially emit perfume in an excessive quantity and do not guarantee its constant and even diffusion throughout the passenger compartment.

To at least partially obviate these problems, there are known deodorising devices which, as a diffuser, exploit the air stream flowing from an air vent of the air-conditioning system of the vehicle.

These deodorising devices comprise an outer casing or bottle designed to be fitted onto an air vent of the vehicle, said outer casing being provided with at least one rear inlet aperture for the inflow of an air stream, and one outlet aperture or outlet slit for the outflow of the deodorazing or perfuming air stream.

Inside the casing or bottle, the flow of incoming air laps a deodorising and/or perfuming substance in the liquid state, for example soaked into a diffusing medium, which is drawn regularly, for example by means of a small pump, from a container secured to the outer casing of the device.

While on the one hand a deodorising device of this kind, makes it possible to achieve a more constant and even distribution of the perfuming or deodorazing substance in the passenger compartment, it presents further drawbacks in that the liquid deodorising and/or perfuming substance is liable to leak from the container. In the event of being inadvertently poured onto the internal upholstery of the vehicle, the latter could require a laborious cleaning operation or suffer damage which can only be remedied by replacing the upholstery itself.

Moreover, since the deodorising device usually is fitted in an easily accessible position, whenever it may be grasped by small children, the possible leakage of the liquid deodorising and/or perfuming substance from the container constitutes a health hazard, in the event for example of the deodorising substance being swallowed or coming into contact with the eyes.

### OBJECT OF THE INVENTION

Object of this invention is to provide a device for emanating a deodorising and/or perfuming substance in a passenger compartment of a vehicle, which is structurally simple and inexpensive, and which allows a constant and even diffusion of the perfuming and/or deodorising substance in the passenger compartment, while at the same time remaining safe to use even in the event of it being inadvertently overturned or imprudently handled.

### BRIEF DESCRIPTION OF THE INVENTION

All the above can be achieved by means of a device for the diffusion of a deodorising and/or perfuming substance, applicable to an internal air vent of a vehicle, characterised by comprising:
- an outer casing;
- at least one air inlet aperture on a rear wall of the casing, for the inflow of an air stream from said air vent, and at least one air outlet aperture on a side wall of the casing, said inlet and outlet apertures defining a flow path for the air stream inside the casing;
- an inner container in said casing, for a deodorising and/or perfuming gel substance in a solid form, said inner container having a open fronted end facing said air flow path; and
- manually operable control means for gradual withdrawal of the deodorising/perfuming gel substance from the inner container for its progressive exposure to the air flow.

### BRIEF DESCRIPTION OF THE DRAWINGS

These and further features according to this invention, will be more clearly evident from the following description with reference to the accompanying drawings, in which:
- Fig. 1 shows a front view of a deodorising/perfuming device according to a first embodiment;
- Fig. 2 shows a side view of the deodorising/perfuming device of Fig. 1;

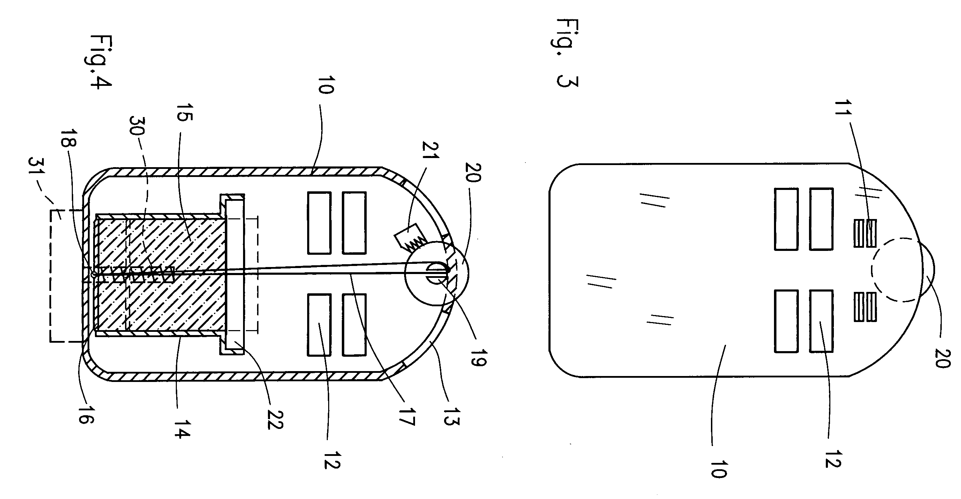

- Fig. 3 shows a rear view of the deodorising/perfuming device of Fig. 1;
- Fig. 4 shows a longitudinal cross-sectional view of the deodorising/perfuming device of Fig. 1;
- Fig. 5 shows a longitudinal cross-sectional view of a deodorising/perfuming device according to a further embodiment.
- Fig. 6 shows a further embodiment of the device.

### DETAILED DESCRIPTION OF THE INVENTION

The general features of this invention will be more illustrated hereunder by means of some embodiments.

The device for diffusing a deodorising and/or perfuming substance in the passenger compartments of motor vehicles, according to a first embodiment shown in the figures from 1 to 4, comprises an outer casing 10, in plastic material, which can be removably fitted onto an internal air vent of a vehicle by any type of fastening means, for example by a clip 11 provided on a rear side of the outer casing 10 of the device.

The outer casing 10 on a rear wall is provided with at least one air-inlet aperture 12 to allow the inflow of an air stream from the air vent of the vehicle, and at least one air outlet aperture or slit 13 on a side wall to allow the outflow of the deodorising and perfumed air stream from the device; the air-inlet and air-outlet apertures define an air-flow path inside the casing 10.

A open fronted container 14 for a deodorising and/or perfuming substance in the form of a gel substance 15 in a solid state, is provided inside the casing 10; more precisely, as shown in figure 4, the open fronted side of the container 14 is facing the air flow path, for example the air outlet aperture, to gradually expose the gel substance to the wirling action of the air flow. The deodorising and/or perfuming substance is in the form of a solid gel substance suitable to maintain its solid state at heated conditon also, up to 80°C; this makes it possible to achieve the scope of this invention, in that, thanks to its solidified state, the gel cannot leak from the device, maintaining at the same time excellent characteristics of emission of perfumed particles, without presenting particular drawbacks.

In figure 4, the inner container 14 for the gel 15 comprises a cylindrical body having a open fronted side and a bottom wall 16 slidably movable inside the container 14 to allow a withdrawal and a progressive exposure of the gel substance 15 to the air stream flowing through the outer casing 10.

The bottom wall 16 is operatively connected to manually operable control means for adjusting the gradual withdrawal of the deodorising/perfuming gel substance 15 from the container 14.

Said control means may be of any suitable type and differently positioned on the casing 10; in the example of figure 4, the manually operated control means comprise a pulling wire 17 having one end connected at 18 to the bottom wall 16 of the gel container, and whose other end is connected to be wound around a pivot pin 19 of a manually operable control wheel 20; the control wheel 20 is rotatably supported inside the outer casing 10, to partially protrude from the upper side

Preferably, the pivot 19 is provided with a cross slit for the passage of the pulling wire 17 to enable it to be wound by a gradual rotation of the control wheel 20.

During use, by turning the control wheel 20 it is possible to adjust the position of the bottom wall 16 inside the container 14 to gradually expose the gel substance 15 to the stream of air flowing into the outer casing 10; depending upon the requirements of deodorising/perfuming the passenger compartment, it is possible therefore to move the gel substance 15 until the desired olfactory effect is perceived. Moreover, the gradual consumption of the gel substance 15 can be compensated by adjusting the position of the bottom wall 16 from time to time by means of the control wheel 20.

To prevent the bottom wall 16 from moving back, the device is provided with braking means 21, to prevent the backward rotation of the control wheel 20 while allowing free rotation in forward direction.

Since the gel substance 15, upon depletion, becomes pulverised, the container 14 is provided, at the open end which is opposite to the bottom wall 16, with a bell-shaped or enlarged wall portion 22, designed to collect the depleted pulverised gel 15.

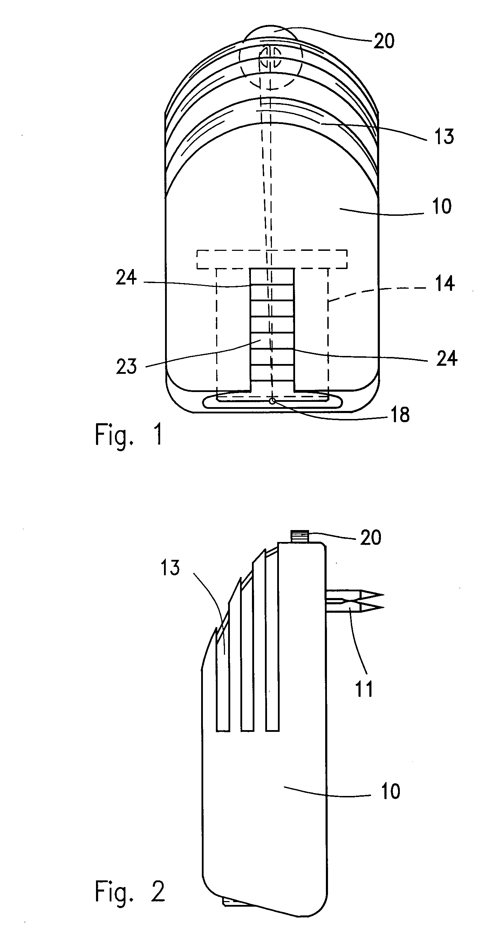

The device may also comprise means for provide an indication of the consumption of the deodorising gel substance 15, made visible through a control window 23 provided on the front wall of the outer casing 10; said means for indicating the consumption of the gel substance 15, according to the embodiment of figure 1 comprises a side wall of the container 14 made of transparent plastic material to allow the visualisation and the position of the gel substance 15 inside the container 14.

Along the transparent lateral wall of the container 14 a plurality of display marks 24 are provided to indicate the position of the deodorising gel 15.

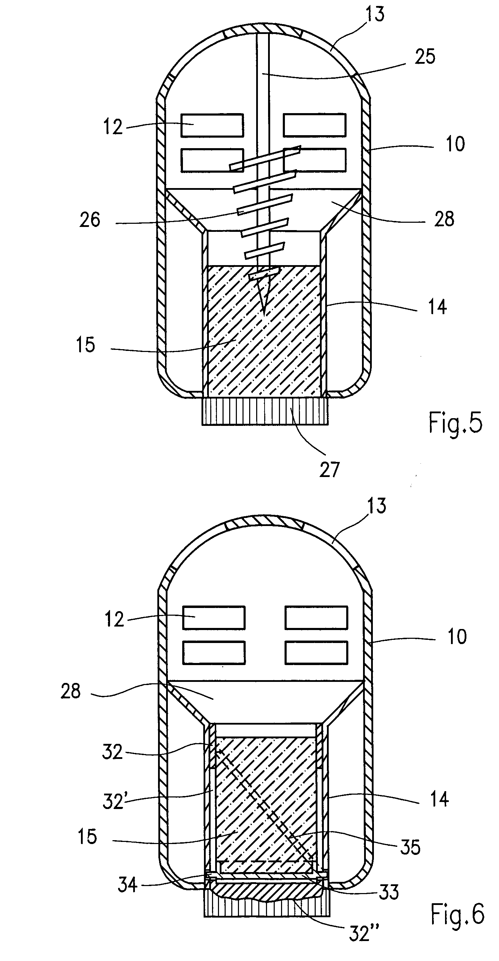

A second embodiment of the device is shown in figure 5, in which the same reference numbers have been used to indicate similar or equivalent parts.

In this second embodiment, which presents more simple and economical structural features, the outer casing 10 rotatingly supports the container 14 for the deodorising/perfuming gel substance 15. In addition, the control means for adjusting the withdrawal of the gel substance 15 from the container 14, comprises a longitudinal rod 25 fastened to and pending from the upper side of the outer casing 10; the rod 25 is provided with a thread end portion 26 designed to penetrate into and cause a progressive withdrawal of the gel substance 15, by rotation of the container 14. Preferably, the thread end 26 is in the form of a corkscrew conically shaped, while the rotation of the container 14 can be made by acting on a control wheel 27 secured to the bottom side of the container 14.

The container 14 for the deodorising/perfuming gel substance 15 also comprises a bell-shaped upper portion 28 having a conical shape, facing the air-flow path, to allow the passage of the rod 25 and the collection of the pulverised gel 15.

With regard to the first embodiment of figure 4, the control means for adjusting the position of the bottom wall 16 with respect to the container 14 may be in the form of a manually operable screw type control means.

For example as shown in phantom lines in figure 4, the screw type control means can comprise a control screw 30 rotatingly supported by the outer casing 10, to screwingly engage the bottom wall 16 through a threaded central hole; the rotation of the control screw may be again made by means of an appropriate control wheel 31 to cause the sliding of the wall 16 and the consequent protrusion of the gel substance 15 from the container 14.
According to another embodiment shown in figure 6, the screw type control means comprise two side pegs 34 of a cup shaped support member 33 for the gel substance, which radially extends through longitudinal slits 32' of a rotating bush 32 inside a cylindrical container 14; the side pegs 34 engage with a screw thread 35 on the internal wall of the container 14; consequently, by acting on the control portion 32" to rotate the bush 32, the cup shaped support member 33 for the deodorising gel substance 15 is made to rotate and slide, allowing the gel substance to be gradually exposed to the air stream flowing into the outer casing 10.

What has been described and shown with reference to the accompanying drawings, has been given purely by way of example in order to illustrate the general features of the invention, as well some of its preferential embodiments; consequently, other modifications and variations to the device for diffusing a deodorising and/or perfuming substance in the passenger compartment of motor vehicles are possible without thereby departing from the scope of the claims.

## Claims

1. Device for diffusing a deodorising and/or perfuming substance (15) applicable to an internal air vent of a vehicle, **characterised by** comprising:
- an outer casing (10);
- at least one air inlet aperture (12) on a rear wall of the casing (10) for the inflow of an air stream from said air vent, and at least one air outlet aperture(13) on a side wall of the casing (10) said inlet and outlet apertures (12, 13) defining a flow path for said air stream;
- an inner container (14) in said casing, for a deodorising and/or perfuming gel substance (15), in a solid form, said inner container (14) having a open fronted end facing said air flow path; and
- manually operable control means for gradual withdrawal of the deodorising/perfuming gel substance (15) from the inner container (14) for its progressive exposure to the air flow.

2. Device for diffusing a deodorising and/or perfuming substance according to claim 1, **characterised in that** said inner container (14) comprises an enlarged wall portion (22, 28) at its open fronted end.

3. Device for diffusing a deodorasing and/or perfuming substance according to claim 1, **characterised by** comprising a gel substance in a solid state up to 80°C.

4. Device for diffusing a deodorising and/or perfuming substance according to claim 1, **characterised in that** the inner container (14) is rotatingly supported by the outer casing (10), and **in that** said control means for adjustably withdrawing the gel substance (15) comprises a control rod (25) inside the outer casing (10), said control rod (25) having a threaded end portion (26) to penetrate and cause a progressive withdrawal of the gel substance (15), by manual rotation of inner container (14).

5. Device for diffusing a deodorising and/or perfuming substance according to claim 1, **characterised in that** said control means for gradual withdrawal of the gel substance (15) comprises a slidable bottom wall (16) of the inner container (14), and manually operable drive means (20, 31, 32') operatively connected to said slidable bottom wall (16).

6. Device for diffusing a deodorising and/or perfuming substance according to claim 5, **characterised in that** the control means comprises a pulling wire (17) fastened to said bottom wall (16), said pulling wire being connected to a manually operable winding up wheel (20).

7. Device for diffusing a deodorazing and/or perfuming substance according to claim 6, **characterized by** comprising braking means (21) for preventing backward rotation of the winding up wheel (20).

8. Device for diffusing a deodorising and/or perfuming substance according to claim 5, **characterized in that** the control means for withdrawal of the gel substance (15) comprises screw type control means (26, 30, 35).

9. Device for diffusing a deodorising and/or perfuming substance according to claim 8, **characterized in that** the screw type control means comprises a control screw (30) rotatingly supported by the outer casing (10), said control screw (30) being screwingly engaged through a threaded hole of the sliding bottom wall (16) of the inner container (14).

10. Device for diffusing a deodorising and/or perfuming substance according to claim 1, **characterized by** comprising means (23, 24) for indicating the consumption of the gel substance (15), and a control window (23) in the outer casing (10), to make visible said gel consumption indicating means.

11. Device for diffusing a deodorising and/or perfuming substance according to claim 9, **characterized in that** said means for indicating the consumption of the gel substance (15) comprise a side wall of the inner container (14) made of transparent material.

12. Device for diffusing a deodorasing and/or perfuming substance according to claim 10, **characterized in that** the means for indicating the consumption of the gel substance (15) comprises visualising marks (24) on the transparent side wall of the inner container (14).
